# EUROPÄISCHE PATENTSCHRIFT

(11) **EP 1 000 919 B1**
(45) Veröffentlichungstag und Bekanntmachung des Hinweises auf die Patenterteilung: **02.05.2002**
(21) Anmeldenummer: 99122475.9
(22) Anmeldetag: 10.11.1999
(51) Int. Cl.: C07C 43/15, C07C 41/06, C07C 47/02, C07C 31/12

(54) **Verfahren zur Herstellung substituierter Butene**
Process for the preparation of substituted butenes
Procédé pour la préparation de butènes substitués

(30) Priorität: 11.11.1998 DE 19852006
(43) Veröffentlichungstag der Anmeldung: 17.05.2000
(73) Patentinhaber: BASF AKTIENGESELLSCHAFT, 67056 Ludwigshafen (DE)
(72) Erfinder: Aron, Maik, 67251 Freinsheim (DE); Böhling, Ralf, 64347 Griesheim (DE); Zehner, Peter, 67074 Ludwigshafen (DE)
(74) Vertreter: Kinzebach, Werner, Dr.

(56) Entgegenhaltungen:
- EP-A- 0 025 240
- WO-A-94/00411
- DE-A- 2 550 902
- DE-A- 2 944 914
- DE-A- 4 400 837
- US-A- 2 922 822

## Beschreibung

Die vorliegende Erfindung betrifft ein Verfahren zur Herstellung substituierter Butene, insbesondere von Alkyloxy-, Aryloxy- oder Aralkyloxybutenen.

Substituierte Butene der Formel I und/oder Formel II 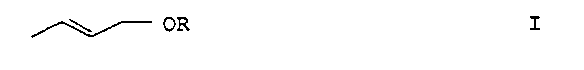 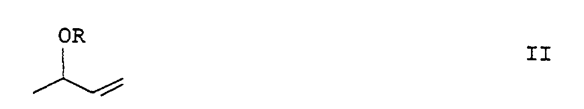 worin der Rest R eine unsubstituierte oder mit einer oder zwei C₁bis C₁₀-Alkoxy- oder Hydroxygruppen substituierte C₂- bis C₂₀-Alkyl- oder Alkenyl, eine C₆- bis C₁₀-Aryl- oder eine C₇- bis C₁₁-Aralkylgruppe oder die Methylgruppe ist, sind wichtige Zwischenprodukte z.B. bei der Herstellung von n-Butyraldehyd und/-oder n-Butanol. Sie werden durch Umsetzung von 1,3-Butadien mit einem Alkohol erhalten. Die DE 4400 837 beschreibt ein Verfahren zur Herstellung von n-Butyraldehyd und/oder n-Butanol bei dem die substituierten Butene der Formel I bzw. II zum Enolether der Formel III 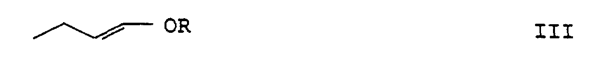 isomerisiert werden und aus diesem Enolether durch dessen Umsetzung mit Wasserstoff und Wasser oder Wasser in Gegenwart eines Übergangsmetallelement-Katalysators n-Butyraldehyd und/oder n-Butanol erzeugt wird.

Die DE 4400 837 empfiehlt, dass zur Umsetzung von Butadien mit dem Alkohol feste Brönsted-Säuren, insbesondere organische Ionenaustauscher, verwendet werden sollen, die in einem Festbett angeordnet und von der flüssigen Reaktionsmischung in der Sumpfoder Rieselfahrweise durchströmt werden.

Es hat sich gezeigt, dass bei der in der DE 4400 837 vorgeschlagenen Konzeption der Druckabfall entlang des Katalysatorbetts mit zunehmender Betriebsdauer steigt. Zur Beherrschung des steigenden Druckabfalls muß der Katalysator daher im Abstand von wenigen Wochen ausgetauscht werden. Die Untersuchung des entnommenen Katalysators zeigte, dass die Ionenaustauscherkugeln in Gegenwart des Reaktionsmediums quellen. Hierdurch treten Kräfte in axialer und radialer Richtung des Reaktors auf. Vor allem die Ionenaustauscherkugeln im unteren Teil des Reaktors können diesen Kräften nicht ausweichen und erleiden eine Deformation. Das Katalysatorbett wird auf diese Weise verdichtet, und sein Lückenvolumen sinkt. Für einen weiteren Durchsatz des Reaktionsmediums muß ein zunehmend höherer Druck aufgebracht werden.

Die Untersuchungen zeigten außerdem Ablagerungen eines gummiartigen Polymeren, das große Teile der Oberfläche der Katalysatorteilchen bedeckte. Die Ablagerungen führen zu einer erheblichen Desaktivierung des Ionenaustauschers. Es handelt sich bei den Ablagerungen vermutlich um Polymerisationprodukte des Butadiens.

Der vorliegenden Erfindung liegt daher die Aufgabe zugrunde, ein Verfahren anzugeben, das eine Verdichtung des Katalysatorbettes und Polymerablagerungen auf der Katalysatoroberfläche verhindert.

Erfindungsgemäß wird diese Aufgabe durch ein Verfahren zur Herstellung substituierter Butene der Formel I und/oder Formel II, 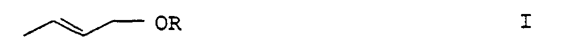 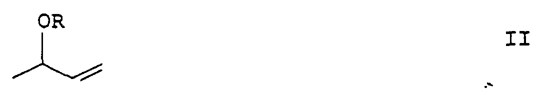 worin der Rest R eine unsubstituierte oder mit einer oder zwei C₁bis C₁₀-Alkoxy- oder Hydroxygruppen substituierte C₂- bis C₂₀-Alkyl- oder Alkenyl-, eine C₆- bis C₁₀-Aryl- oder eine C₇- bis C₁₁-Aralkylgruppe oder die Methylgruppe ist, durch Umsetzung von 1,3-Butadien mit einem Alkohol der Formel ROH, in der der Rest R die angegebene Bedeutung besitzt, in Gegenwart eines sauren, teilchenförmigen, im Reaktionsmedium unlöslichen Katalysators gelöst, das dadurch gekennzeichnet ist, dass das Inkontaktbringen des Butadiens, des Alkohols und des Katalysators in einem von unten angeströmten, oberhalb des Lockerungspunktes betriebenen Fließbettreaktor erfolgt.

Im von unten, d.h. entgegen der Richtung der Schwerkraft, angeströmten Fließbett können die Katalysatorteilchen quellen, ohne zu verpressen. Die gegenseitige Reibung der Katalysatorteilchen verhindert vermutlich die Bildung von Polymerablagerungen.

Fig. 1 zeigt die relative Katalysatoraktivität in Abhängigkeit von der produzierten Produktmenge im Festbettbetrieb und im erfindungsgemäßen Fließbettbetrieb.

Die Figuren 2 und 3 zeigen rasterelektronenmikroskopische Aufnahmen von Ionenaustauscherkugeln nach 35 Tagen Einsatz im Festbettbetrieb (Fig. 2a, 2b) beziehungsweise 20 Tagen Einsatz im Fließbettbetrieb (Fig. 3a, 3b).

1,3-Butadien ist eine Grundchemikalie, die in großen Mengen in Steamcrackern erzeugt und aus dem C₄-Schnitt des Steamcrackers extraktiv, beispielsweise mittels N-Methylpyrrolidon, isoliert wird.

1,3-Butadien wird in Gegenwart eines Katalysators mit dem Alkohol ROH gemäß Gleichung (1) 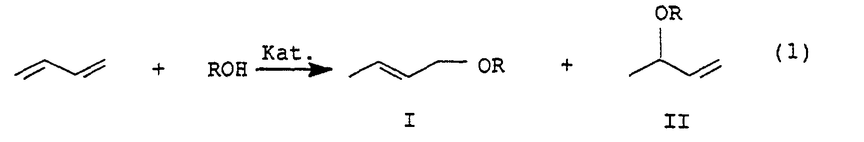 zum 1,4-Addukt der Formel I und dem 1,2-Addukt der Formel II umgesetzt. Im entstandenen 1,4-Addukt I kann die Doppelbindung sowohl in der cis- als auch trans-Form vorliegen. Die Addukte I und II entstehen dabei je nach Reaktionsbedingungen und angewandtem Katalysator im Allgemeinen in einem Molverhältnis von 1:1 bis 1:3.

Die Art des in der Umsetzung eingesetzten Alkohols ROH ist in der Regel für das Verfahren nicht kritisch. Es können sowohl primäre als auch sekundäre Alkohole verwendet werden, bevorzugt werden aber primäre Alkohole eingesetzt. Es können sowohl aliphatische, cycloaliphatische, aromatische als auch araliphatische Alkohole benutzt werden, vorzugsweise finden aliphatische und araliphatische Alkohole Anwendung. Im Allgemeinen werden Alkohole ROH im erfindungsgemäßen Verfahren verwendet, in denen der Rest R eine C₁- bis C₂₀-Alkyl-, C₂- bis C₁₀-Alkenyl-, z.B. die But-2-enyl-, vorzugsweise eine C₁- bis C₄-Alkyl-, insbesondere die n-Butylgruppe, eine C₆- bis C₁₀-Aryl-, vorzugsweise die Phenylgruppe oder eine C₇- bis C₁₁-Aralkyl-, vorzugsweise die Benzylgruppe ist. Die Reste R können gegebenenfalls mit Substituenten wie C₁- bis C₁₀-Alkoxy- und/oder Hydroxyl-Gruppen substituiert sein. Als Alkohole ROH können somit auch Diole oder Triole oder Alkoxyalkohole verwendet werden. Da diese Substituenten in der Regel keinen kritischen Einfluß auf die Umsetzung haben, werden vorzugsweise Alkohole ROH mit unsubstituierten Resten R verwendet. Selbstverständlich können auch Alkohole mit einer höheren Anzahl an Kohlenstoffatomen eingesetzt werden, da solche höheren Alkohole in der Regel teurer sind als niedere Alkohole, werden aus wirtschaftlichen Gründen niedere Alkohole bevorzugt verwendet.

Die Umsetzung erfolgt in Gegenwart eines sauren, teilchenförmigen, im Reaktionsmedium unlöslichen Katalysators. Die Verwendung organischer Kationenaustauscher ist dabei bevorzugt.

Unter organischen Kationenaustauschern werden pulverförmige, gelartige oder makroporöse, polymere Polyelektrolyte verstanden, die Brönsted-acide funktionelle Gruppen, wie Sulfonsäure-, Phosphonsäure- oder Carboxylgruppen auf einer polymeren Matrix tragen, beispielsweise sulfonierte Phenol-Formaldehyd-Harze, sulfonierte Styrol-Divinylbenzol-Copolymere, sulfoniertes Polystyrol, Poly(perfluoralkylen)sulfonsäuren oder sulfonierte Kohlen. Zweckmäßigerweise kommen die Kationenaustauscher im erfindungsgemäßen Verfahren in ihrer protonierten Form, der sogenannten H⁺-Form, zur Anwendung. Als geeignete organische Kationenaustauscher seien beispielhaft die Handelsprodukte Amberlite® 200, Amberlite® IR 120, Amberlite® IR 132 E, Lewatit® SC 102, Lewatit® SC 104, Lewatit® SC 108, Lewatit® SPC 108, Lewatit® SPC 112, Lewatit® SPC 118 und Amberlyst® 15, Amberlyst® 35 wet, Amberlyst® 38 wet, Amberlyst® CSP 2, Amberlyst® CSP 3, und Purolite® CT 175 genannt.

Die Ionenaustauscher weisen vorzugsweise einen Gehalt an sauren Gruppen von 4 bis 6, vorzugsweise 5 bis 5,5 eq/kg auf.

Anstelle organischer, saurer Kationenaustauscher können im erfindungsgemäßen Verfahren auch feste, Brönsted-acide wirkende anorganische Feststoffe eingesetzt werden, beispielsweise Zeolithe, wie β-Zeolithe oder Y-Zeolithe in der H⁺-Form, Bleicherden, wie Bentonite, Montmorillonite oder Attapulgite, nichtzeolitische Molekularsiebe auf Phosphatbasis, wie sie beispielsweise Gegenstand von US-A 4 440 871, US-A 4 310 440, US-A 4 567 029, US-A 4 554 143, US-A 4 500 651, EP-A 158 976, EP-A 158 349, EP-A 159 624 sind, sowie saure oder säureimprägnierte Metalloxide, deren Herstellung z.B. in US-A 4 873 017 beschrieben ist. Bevorzugte Brönsted-acide anorganische Feststoffe sind β-Zeolithe oder Y-Zeolithe in der H⁺-Form, insbesondere β-Zeolithe in der H⁺-Form. β-Zeolithe sind z.B. nach dem Verfahren von US A 4 891 458 erhältlich.

Die Erfindung beruht auf der Verwendung eines vertikalen Fließbettreaktors zum Inkontaktbringen des Butadiens, des Alkohols und des Katalysators. Das erfindungsgemäße Verfahren wird mit einem flüssigen Reaktionsmedium durchgeführt, das vorzugsweise im Wesentlichen homogen ist. Der Fließbettzustand liegt vor, wenn die Lineargeschwindigkeit eines Fluids in einer von unten angeströmten Schüttung über die sogenannte Auflockerungsgeschwingigkeit hinaus erhöht wird, so dass die Einzelteilchen in Schwebe gehalten werden und homogen im Fluid suspendiert sind. Am Lockerungspunkt befindet sich die Schüttung im Zustand der beginnenden Bettausdehnung. Die experimentelle Bestimmung des Lockerungspunktes erfolgt durch Messung des Druckverlustes Δp im Fließbett in Abhängigkeit von der Querschnittsbelastung. Der Lockerungspunkt ergibt sich dann als der Schnittpunkt zwischen der extrapolierten Festbettkennlinie und der Linie konstanten Druckabfalls im Fließbett.

Die in vertikaler Richtung gemessene Höhe l₀ der Schüttung des Katalysators im Ruhezustand dehnt sich im Betrieb des Reaktors auf die Höhe L aus. Die auf l₀ bezogene Differenz (L-l₀) wird als sogenannte Bettausdehnung bezeichnet. Es ist bevorzugt, dass die Bettausdehnung mindestens 5%, vorzugsweise 7 bis 50%, insbesondere 12 bis 15% bezogen auf den Ruhezustand, beträgt.

Das auf den Reaktorquerschnitt (in m²) und die Zeit (in h) bezogene Volumen des durch die Schüttung geführten Reaktionsmediums wird als Querschnittsbelastung des Reaktors bezeichnet. Diese beträgt unter Reaktionsbedingungen vorzugsweise 5 bis 100, insbesondere 7 bis 60, besonders bevorzugt 10 bis 30 m³/m²·h.

Zur Erreichung der zum Fließbettbetrieb erforderlichen Querschnittsbelastung des Reaktors kann es zweckmäßig sein, einen Teil des Reaktoraustrags zurückzuführen. Das Rückführverhältnis hängt stark von der jeweiligen Reaktorgeometrie ab.

Für ein vorteilhaftes Fließverhalten weist der Katalysator vorzugsweise eine bestimmte Dichte (Schüttdichte) auf. Der Katalysator weist vorzugsweise eine Dichte von 700 bis 900 g/l, insbesondere von 720 bis 850 g/l auf. Die Katalysatorteilchen weisen vorzugsweise außerdem einen bestimmten Durchmesser auf, der im Allgemeinen 0,2 bis 1,5 mm, insbesondere 0,4 bis 1,2 mm beträgt.

Es wurde überraschenderweise gefunden, dass eine weitergehende Optimierung des erfindungsgemäßen Verfahrens durch die Kontrolle der Porengrößenverteilung des Katalysators möglich ist. Es erwies sich, dass die Reaktiongeschwindigkeit gesteigert und Nebenreaktionen zurückgedrängt werden, wenn der Katalysator eine poröse Matrix umfasst, die ein Maximum der Porenradienverteilung, bestimmt nach BET, im Bereich von 20 bis 100 nm, vorzugsweise 40 bis 80 nm, aufweist. Die Porenradienverteilung kann mehrere Maxima aufweisen. Für die vorliegenden Zwecke ist es ausreichend, wenn ein Maximum (neben gegebenenfalls weiteren vorhandenen Maxima) der Porenradienverteilung im angegebenen Bereich liegt. Die Bestimmung der Porenradienverteilung erfolgt zweckmäßigerweise nach der Methode nach Brunauer, Emmett und Teller (BET-Methode). Hierbei wird das Volumen des Stickstoffgases, das bei -196°C in Abhängigkeit vom angewandten Druck auf dem Prüfgegenstand adsorbiert wird, gemessen. Die Zahl der Poren mit dem jeweiligen Durchmesser wird gegen den Durchmesser aufgetragen. Im Einzelnen wird auf DIN 66131, 1993, Beuth Verlag GmbH, Berlin verwiesen. Zur Durchführung der Messung ist ein Micromeritics ASAP 2400 Gerät geeignet.

Der Einfluß der Porengröße beruht vermutlich auf einer Kontrolle der Zugänglichkeit der aktiven Zentren für die Reaktionsteilnehmer. Organische Kationenaustauscher, deren polymere Matrix die angegebene Porengrößenverteilung aufweist, sind besonders bevorzugt.

Das Molverhältnis Alkohol/1,3-Butadien kann im erfindungsgemäßen Verfahren aus einem weiten Bereich gewählt werden. Im Allgemeinen wird im Zulauf ein Molverhältnis Alkohol ROH/1,3-Butadien von 0,5:1 bis 15:1 vorzugsweise von 1:1 bis 5:1 und besonders bevorzugt von 1,8:1 bis 3,5:1 angewandt. Die Umsetzung des Alkohols ROH mit 1,3-Butadien wird im Allgemeinen bei Temperaturen von 20 bis 130°C, vorzugsweise von 50 bis 120°C, insbesondere von 75 bis 100°C und bei einem Druck von im Allgemeinen 3 bis 100 bar, vorzugsweise von 5 bis 50 bar, insbesondere von 7 bis 20 bar, vorgenommen. Zweckmäßigerweise wird der Druck so gewählt, dass das 1,3-Butadien bei der angewandten Reaktionstemperatur flüssig ist. Die Anwendung eines höheren Drucks ist möglich.

Der Zusatz eines Lösungsmittels zum Reaktionsgemisch ist möglich, im Allgemeinen aber nicht erforderlich, da sowohl der eingesetzte Alkohol als auch die Addukte I und II auch als Lösungsmittel fungieren können.

Der Wassergehalt des Reaktionsgemisches wird vorzugsweise kontrolliert. Ein zu hoher Wassergehalt führt zu einer Hydratisierung der aktiven Zentren des Katalysators und behindert so den Zutritt von Alkohol und/oder 1,3-Butadien. Vorzugsweise beträgt die Wasserkonzentration höchstens 3 Gew.-%, insbesondere 0,1 bis 2 Gew.-%.

In einer bevorzugten Ausführungsform handelt es sich bei dem Alkohol ROH um n-Butanol. In diesem Fall hat das Reaktionsgemisch am Reaktorausgang im Allgemeinen folgende Zusammensetzung:
1 bis 30 Gew.-%, vorzugsweise 3 bis 20 Gew.-%, insbesondere 5 bis 15 Gew.-%, 1,3-Butadien,
4 bis 40 Gew.-%, vorzugsweise 6 bis 35 Gew.-%, insbesondere 8 bis 30 Gew.-% 1-Butoxybuten,
4 bis 60 Gew.-%, vorzugsweise 8 bis 45 Gew.-%, insbesondere 10 bis 35 Gew.-%, 3-Butoxybuten,
20 bis 80 Gew.-%, vorzugsweise 30 bis 70 Gew.-%, insbesondere 40 bis 65 Gew.-% n-Butanol und
0 bis 10 Gew.-%, vorzugsweise 0,1 bis 3 Gew.-%, insbesondere 0,2 bis 1,2 Gew.-% Wasser.

Der Reaktionsaustrag des erfindungsgemäßen Verfahrens enthält im Allgemeinen neben nicht umgesetztem 1,3-Butadien die Addukte der Formeln I und II sowie gegebenenfalls geringe Mengen an Reaktionsnebenprodukten, wie Alkoxyoctadiene, Octatrien, Vinylcyclohexen, Alkoxydodecatriene, Dodecatetraen, Dialkoxyocten und Dialkoxybutan. Unumgesetztes Butadien und unumgesetzter Alkohol aus dem Reaktoraustrag werden zweckmäßigerweise in das Verfahren zurückgeführt. Dabei muß in der Regel mitgeführtes Wasser, das aus Nebenreaktionen stammt, ausgeschleust werden.

Das zur Herstellung von n-Butyraldehyd und/oder n-Butanol benötigte Addukt ist das 1-Alkoxy-buten-2 der Formel I. Daher wird das Addukt II zunächst aus dem im Reaktionsaustrag enthaltenen isomeren Addukt I abgetrennt.

Das vom gewünschten Addukt abgetrennte Addukt II kann dann in das erfindungsgemäße Verfahren zurückgeführt werden. Die Rückführung des Addukts II in das erfindungsgemäße Verfahren bewirkt die Isomerisierung des Addukts II zum Addukt I in dieser Verfahrensstufe und führt letztendlich zur Unterdrückung der Neubildung des unerwünschten Addukts II, so dass bei Anwendung dieser Kreislauffahrweise in der Gesamtbilanz dieses Kreisprozesses praktisch nur das erwünschte Addukt I, nicht jedoch dessen Isomer II gebildet wird.

Im weiteren Verlauf des Verfahrens zur Herstellung von n-Butyraldehyd und/oder n-Butanol wird das Addukt I katalytisch zum Enolether der Formel III isomerisiert, der anschließend katalytisch in Gegenwart von Wasser zu n-Butyraldehyd hydrolysiert und/oder katalytisch in Gegenwart von Wasser und Wasserstoff in n-Butanol umgewandelt wird (siehe Gleichung (2)). Diese Teilreaktionen können im Verfahren wahlweise sukzessive in zwei Verfahrensstufen oder sukzessive in einem einzelnen Reaktor oder besonders vorteilhaft einstufig, in einer einzigen Verfahrensstufe durchgeführt werden. Beide Teilreaktionen können sowohl in der Gasphase als auch in flüssiger Phase stattfinden. Als Katalysatoren sind Übergangselement-Katalysatoren geeignet.

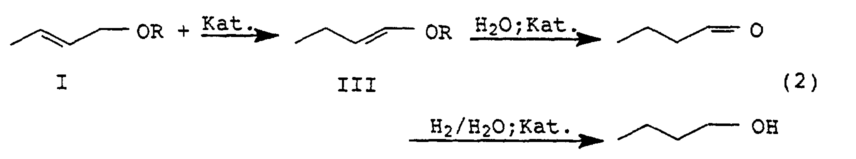

Bezüglich der Reaktionsbedingungen und der anwendbaren Katalysatoren für die weitere Umsetzung der substituierten Butene I und/-oder II zum Enolether III und weiter zu n-Butyraldehyd und/oder n-Butanol wird auf die DE 4 400 837 verwiesen. Es ist bevorzugt, dass man die Isomerisierung und die weitere Umsetzung in einer Verfahrensstufe vornimmt. Als Katalysatoren sind beispielsweise ein- oder mehrzähnige Phosphin- oder Phosphit-Komplexe eines Elements aus den Gruppen Ib, VIb, VIIb und/oder VIIIb des Periodensystems der Elemente geeignet. Weiter sind Komplexe stickstoffhaltiger Chelat-Liganden, wie Bipyridin- oder Phenanthrolin-Liganden, mit einem Element der Gruppe Ib, VIb, VIIb und/oder VIIIb des'Periodensystems der Elemente geeignet. Ferner brauchbar sind Salze eines Elements aus der Gruppe Ib, VIb, VIIb und/oder VIIIb des Periodensystems der Elemente. Schließlich eignen sich auch Aquo-, Ammin-, Halogeno-, Cyano-, Carbonyl-, Amino- und/oder Acetylacetonato-Komplexe eines Elements aus der Gruppe Ib, VIb, VIIb und/oder VIIIb des Periodensystems der Elemente.

Die Erfindung wird durch die folgenden Beispiele näher veranschaulicht:

### Beispiel 1

n-Butanol wurde in Gegenwart eines sauren Ionenaustauschers an 1,3-Butadien addiert. Die Umsetzung erfolgte im Fließbettbetrieb in einem Glasreaktor. Die Versuchsparameter waren wie folgt:

| | |
|---|---|
| Temperatur | 80°C |
| Druck | 10 bar |
| Zulaufstrom | 13 g/h |
| Zulaufzusammensetzung | 80 Gew.-% Butanol, 19,9 Gew.-% Butadien, 0,1 Gew.-% H₂O |
| Reaktordurchmesser | 20 mm |
| Betthöhe (nicht expandiert) | 135 mm |
| Katalysator | Ionentauscher Purolite CT-175 |
| Kugeldurchmesser | 0,3 - 1,2 mm |
| Kreislaufmenge | 3,1 kg/h |
| Flüssigkeitsbelastung | 12,4 m³/m²·h |
| Bettausdehnung | 10 mm |

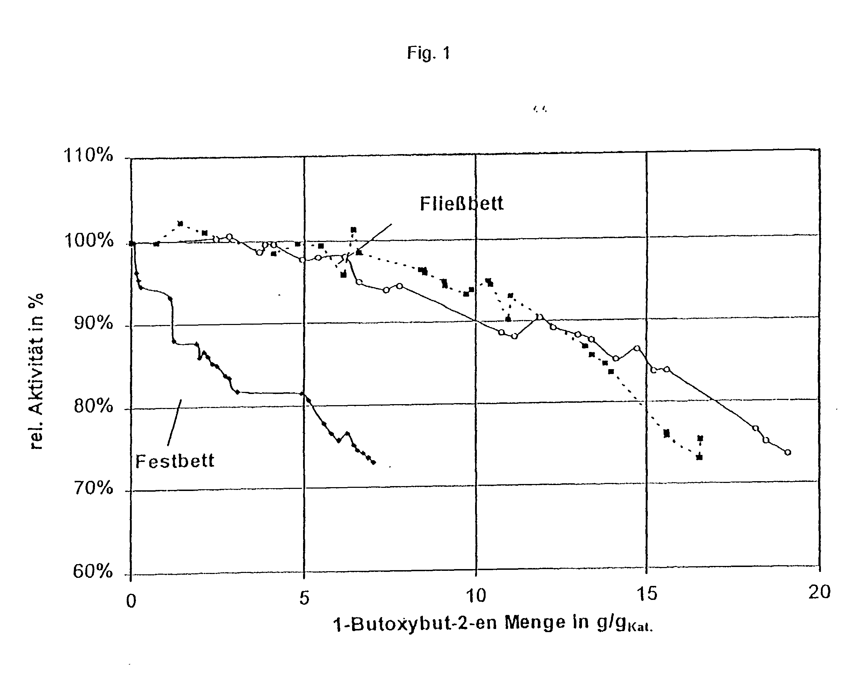

Der eingesetzte Ionenaustauscher lag als Kugeln mit einem Durchmesser von 0,3-1,2 mm vor. Seine Dichte betrug 750-850 g/l, der Gehalt an sauren Gruppen 5 eq/kg. Die Porenradienverteilung wies ein Maximum bei 45 nm auf. Während des Versuchszeitraumes von 600 h wurde kein Anstieg des Druckverlustes beobachtet. Die relative Aktivität, bezogen auf die Anfangsaktivität (die gleich 100 gesetzt wurde), des Katalysators in Abhängigkeit von der seit Versuchsbeginn produzierten Menge an 1-Butoxybut-2-en ist in Figur 1 aufgetragen (leere Kreise bzw. ausgefüllte Quadrate; die Meßwerte stammen aus zwei Versuchen). Dabei wurde der Zulaufstrom so variiert, dass stationäre Reaktandenkonzentrationen erhalten wurden. Mit zunehmender Versuchsdauer mußte der Zulaufstrom aufgrund der abnehmenden Katalysatoraktivität reduziert werden.

### Beispiel 2

Beispiel 1 wurde wiederholt, wobei die Versuchsparameter wie folgt waren:

| | |
|---|---|
| Temperatur | 80°C |
| Druck | 10 bar |
| Zulaufstrom | 13 g/h |
| Zulaufzusammensetzung | 80 Gew.-% Butanol, 19,9 Gew.-% Butadien, 0,1 Gew.-% H₂O |
| Reaktordurchmesser | 20 mm |
| Betthöhe (nicht expandiert) | 137 mm |
| Katalysator | Ionentauscher Amberlyst CSP 2 |
| Kugeldurchmesser | 0,4 - 1,2 mm |
| Kreislaufmenge | 5,6 kg/h |
| Flüssigkeitsbelastung | 22,4 m³/m²·h |
| Bettausdehnung | 20 mm |

### Vergleichsbeispiel

Die Addition von n-Butanol an Butadien wurde mit der gleichen Zulaufzusammensetzung und mit dem gleichen Ionentauscher wie im obigen Beispiel 1 durchgeführt, wobei jedoch die Umsetzung im von oben durchströmten Festbettbetrieb erfolgte. Die Versuchsparameter waren im übrigen wie in Beispiel 1 angegeben.

Die relative Aktivität des Katalysators (bezogen auf die Anfangsaktivität, die gleich 100% gesetzt wurde) in Abhängigkeit von der seit Versuchsbeginn produzierten Menge an 1-Butoxybut-2-en ist in Figur 1 (ausgefüllte Rauten) dargestellt. Aus der Figur 1 ist ersichtlich, dass die Desaktivierung des Katalysators im Festbettbetrieb deutlich schneller als im Fließbettbetrieb erfolgt.

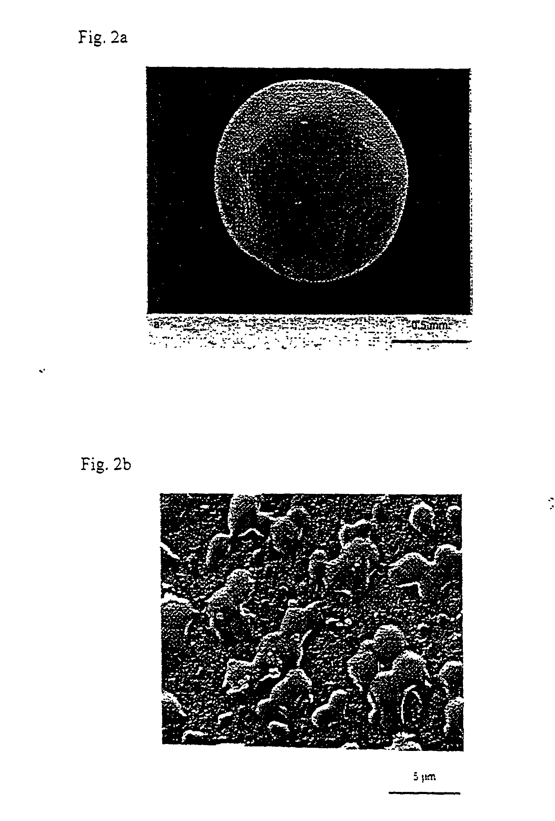

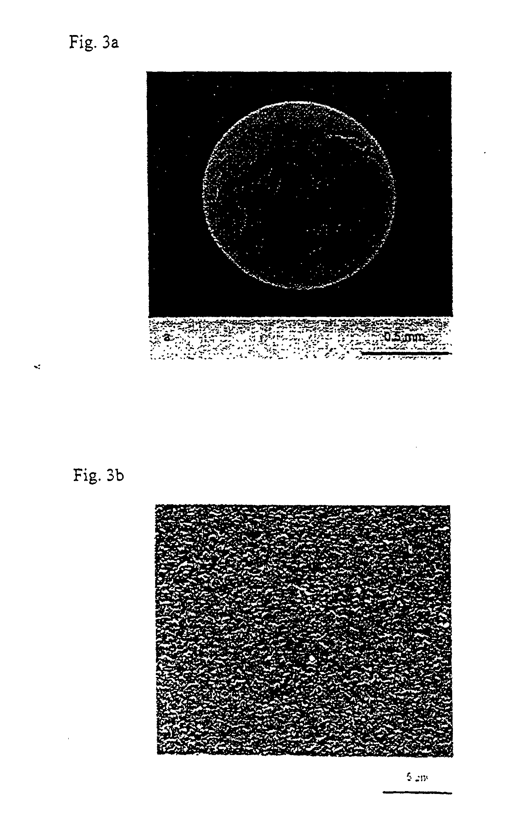

Es wurden ferner rasterelektronenmikroskopische Aufnahmen einer Ionenaustauscherkugel nach 35 Tagen Einsatz im Festbett erstellt (Figur 2a, Figur 2b) bzw. 20 Tagen im Fließbettbetrieb (Figur 3a, Figur 3b). Figur 2a zeigt deutlich die durch das Quellen bedingte Deformation der Ionenaustauscherkugeln, die zu einem Verpressen des Festbetts führt. Außerdem sind in Figur 2b Ablagerungen von gummiartigen Polymeren zu erkennen, die die rasche Desaktivierung des Katalysators verursachen. Figur 3a zeigt die REM-Aufnahme einer Ionenaustauscherkugel nach 20 Tagen im Fließbettbetrieb. Die Ionenaustauscherkugel zeigt keinerlei Spuren von Deformation. Die Ionenaustauscherkugel zeigte nach 20 Tagen im Fließbettbetrieb außerdem kaum Polymerablagerungen (Figur 3b).

## Patentansprüche

1. Verfahren zur Herstellung substituierter Butene der Formel I und/oder Formel II, 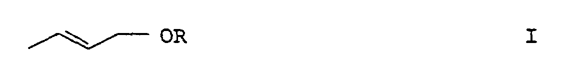 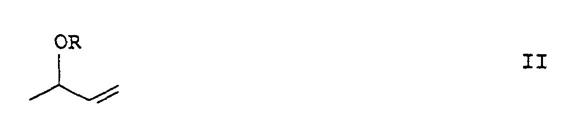 worin der Rest R eine unsubstituierte oder mit einer oder zwei C₁- bis C₁₀-Alkoxy oder Hydroxygruppen substituierte C₂bis C₂₀-Alkyl- oder Alkenyl-, eine C₆- bis C₁₀-Aryl- oder eine C₇- bis C₁₁-Aralkylgruppe oder die Methylgruppe ist, durch Umsetzung von 1,3-Butadien mit einem Alkohol der Formel ROH, in der der Rest R die angegebene Bedeutung besitzt, in Gegenwart eines sauren, teilchenförmigen, im Reaktionsmedium unlöslichen Katalysators, **dadurch gekennzeichnet, dass** das Inkontaktbringen des Butadiens, des Alkohols und des Katalysators in einem von unten angeströmten, oberhalb des Lockerungspunktes betriebenen Fließbettreaktor erfolgt.

2. Verfahren nach Anspruch 1, **dadurch gekennzeichnet, dass** die Bettausdehnung im Betrieb des Reaktors bezogen auf den Ruhezustand mindestens 5% beträgt.

3. Verfahren nach Anspruch 1 oder 2, **dadurch gekennzeichnet, dass** die Querschnittsbelastung des Reaktors 5 bis 100 m³/m² h beträgt.

4. Verfahren nach einem der vorhergehenden Ansprüche, **dadurch gekennzeichnet, dass** der Katalysator als Teilchen mit einem Durchmesser von 0,2 bis 1,5 mm vorliegt.

5. Verfahren nach einem der vorhergehenden Ansprüche, **dadurch gekennzeichnet, dass** der Katalysator eine Dichte von 700 bis 900 g/l aufweist.

6. Verfahren nach einem der vorhergehenden Ansprüche, **dadurch gekennzeichnet, dass** es sich bei dem Katalysator um einen organischen Kationenaustauscher handelt.

7. Verfahren nach Anspruch 6, **dadurch gekennzeichnet, dass** der Ionenaustauscher einen Gehalt an sauren Gruppen von 4 bis 6 eq/kg aufweist.

8. Verfahren nach einem der vorhergehenden Ansprüche, **dadurch gekennzeichnet, dass** der Katalysator eine poröse Matrix umfasst, die ein Maximum der Porenradienverteilung, bestimmt nach BET, im Bereich von 20 bis 100 nm aufweist.

9. Verfahren nach einem der vorhergehenden Ansprüche, **dadurch gekennzeichnet, dass** ein Teil des Reaktoraustrags zurückgeführt wird.

10. Verfahren nach einem der vorhergehenden Ansprüche, **dadurch gekennzeichnet, dass** man das substituierte Buten der Formel I in Gegenwart eines Übergangsmetallelement-Katalysators zum Enolether der Formel III 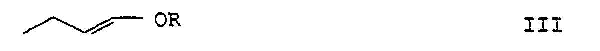 isomerisiert und aus diesem Enolether durch dessen Umsetzung mit Wasserstoff und Wasser oder Wasser in Gegenwart eines Übergangsmetallelement-Katalysators n-Butyraldehyd und/oder n-Butanol erzeugt.

## Claims

1. A process for preparing substituted butenes of the formula I and/or II, 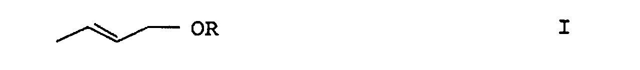 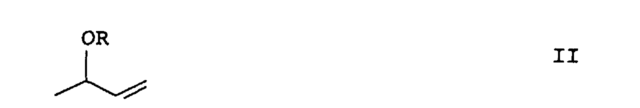 where the radical R is unsubstituted or mono- or di-C₁-C₁₀-alkoxy-substituted or mono- or dihydroxy-substituted C₂-C₂₀-alkyl or alkenyl or is C₆-C₁₀-aryl, C₇-C₁₁-aralkyl or methyl by reacting 1,3-butadiene with an alcohol of the formula ROH in which R is as defined in the presence of an acidic particulate catalyst insoluble in the reaction medium, which comprises contacting the butadiene, the alcohol and the catalyst in a fluidized bed reactor which is flow-approached from below and is operated at above the loosening point.

2. A process as claimed in claim 1, wherein the bed expansion during operation of the reactor is at least 5% relative to the resting state.

3. A process as claimed in claim 1 or 2, wherein the cross-sectional space velocity of the reactor is from 5 to 100 m³/m²·h.

4. A process as claimed in any of the preceding claims, wherein said catalyst is in the form of particles with a diameter of from 0.2 to 1.5 mm.

5. A process as claimed in any of the preceding claims, wherein said catalyst has a density of from 700 to 900 g/l.

6. A process as claimed in any of the preceding claims, wherein said catalyst is an organic cation exchanger.

7. A process as claimed in claim 6, wherein said ion exchanger has an acid group content of from 4 to 6 eq/kg.

8. A process as claimed in any of the preceding claims, wherein said catalyst comprises a porous matrix having a maximum pore radius distribution, determined by the BET method, in the range from 20 to 100 nm.

9. A process as claimed in any of the preceding claims, wherein some of the reactor discharge is recycled.

10. A process as claimed in any of the preceding claims, wherein said substituted butene of the formula I is isomerized in the presence of a transition metal catalyst to give the enol ether of the formula III 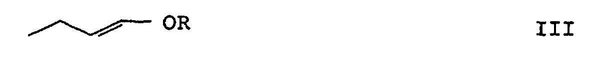 which is reacted with hydrogen and water or water in the presence of a transition metal catalyst to give n-butyraldehyde and/or n-butanol.

## Revendications

1. Procédé de préparation de butènes substitués de formule I et/ou de formule II, 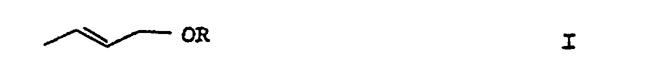 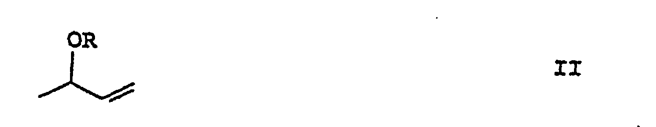 dans laquelle le reste R représente un groupe, non substitué ou substitué par un ou deux groupes hydroxy ou alcoxy en C₁ à C₁₀, alcényle ou alkyle en C₂ à C₂₀, un groupe aryle en C₆ à C₁₀, ou un groupe aralkyle en C₇ à C₁₁, ou encore le groupe méthyle, au moyen d'une réaction de 1,3-butadiène avec un alcool de formule ROH dans laquelle le reste R prend la signification susnommée, en présence d'un catalyseur acide, sous forme de particules, insoluble dans le milieu de réaction, **caractérisé en ce que** l'on met en contact le butadiène, l'alcool et le catalyseur dans un réacteur à lit fluidifié, dans lequel l'alimentation se fait par le bas et que l'on fait fonctionner le réacteur au dessus du point de desserrage.

2. Procédé selon la revendication 1, **caractérisé en ce que** l'extension du lit, lors du fonctionnement du réacteur s'élève à 5% ou plus, par rapport au repos.

3. Procédé selon la revendication 1 ou 2, **caractérisé en ce que** la charge moyenne du réacteur est comprise entre 5 m³/m² et 100 m³/m² par heure.

4. Procédé selon l'une quelconque des revendications précédentes, **caractérisé en ce que** le catalyseur est présent sous forme de particule d'un diamètre compris entre 0,2 mm et 1,5 mm.

5. Procédé selon l'une quelconque des revendications précédentes, **caractérisé en ce que** le catalyseur présente une densité comprise entre 700 g/l et 900 g/l.

6. Procédé selon l'une quelconque des revendications précédentes, caractérisé qu'en ce qui concerne le catalyseur il s'agit d'un échangeur de cations organique.

7. Procédé selon la revendication 6, **caractérisé en ce que** l'échangeur d'ions présente une teneur en groupes acides comprise entre 4 éq/kg et 6 éq/kg.

8. Procédé selon l'une quelconque des revendications précédentes, **caractérisé en ce que** le catalyseur comprend une matrice poreuse dont la distribution du rayon des pores, mesuré selon BET, présente son maximum dans la plage comprise entre 20 nm et 100 nm.

9. Procédé selon l'une quelconque des revendications précédentes, **caractérisé en ce que** l'on recycle une partie du courant de production du réacteur.

10. Procédé selon l'une quelconque des revendications précédentes, **caractérisé en ce que** l'on soumet le butène substitué de formule I à une isomérisation, en présence d'un catalyseur à base de métal de transition élémentaire, pour obtenir un éther énolique de formule III 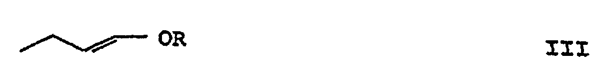 suivi d'une réaction de cet éther énolique avec de l'hydrogène et de l'eau ou avec de l'eau en présence d'un catalyseur à base de métal de transition élémentaire, pour obtenir l'aldéhyde n-butyrique et/ou le n-butanol.
